(19) Europäisches Patentamt
European Patent Office
Office européen des brevets

(11) Veröffentlichungsnummer: **0 031 429**
**B1**

(12) # EUROPÄISCHE PATENTSCHRIFT

(45) Veröffentlichungstag der Patentschrift: **04.07.84**

(51) Int. Cl.³: **C 07 D409/12**, A 61 K 31/415

(21) Anmeldenummer: **80107000.4**

(22) Anmeldetag: **13.11.80**

(54) **2-(N-(2-Fluor-6-bromphenyl)-N-(thiemyl-2-methyl)-amino)-2-imidazolin, dessen Säureadditionssalze, diese enthaltende Arzneimittel und Verfahren zur Herstellung derselben.**

(30) Priorität: **21.12.79 DE 2951601**

(43) Veröffentlichungstag der Anmeldung: **08.07.81 Patentblatt 81/27**

(45) Bekanntmachung des Hinweises auf die Patenterteilung: **04.07.84 Patentblatt 84/27**

(84) Benannte Vertragsstaaten:
**AT BE CH DE FR GB IT LI LU NL SE**

(56) Entgegenhaltungen:
**EP-A- 0 007 428**
**DE-A- 2 208 434**
**DE-A- 2 308 883**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN**
**Postfach 200**
**D-6507 Ingelheim am Rhein (DE)**

(72) Erfinder: **Stähle, Helmut, Dr.**
**Rotweinstrasse 23**
**D-6507 Ingelheim/Rhein (DE)**
Erfinder: **Köppe, Herbert, Dr.**
**Neuweg 72**
**D-6507 Ingelheim/Rhein (DE)**
Erfinder: **Kummer, Werner, Dr.**
**Georg-Scheuing-Strasse 15**
**D-6507 Ingelheim/Rhein (DE)**
Erfinder: **Kobinger, Walter, Prof. Dr.**
**Belghofergasse 27**
**A-1120 Wien (AT)**
Erfinder: **Stockhaus, Klaus, Dr.**
**Pfarrer-Heberer-Strasse 35**
**D-6530 Bingen/Rhein (DE)**

**EP 0 031 429 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Aus der DE-A-2 308 883 sind substituierte 2-(N)Thienyl-N-phenyl-amino)-2-imidazoline bekannt, die eine analgetische Wirksamkeit aufweisen.

Es wurde nun gefunden, daß ein bisher nicht offenbartes Derivat dieser Verbindungsreihe stark analgetische Eigenschaften besitzt.

Die Erfindung betrifft 2-[N-(2-Fluor-6-bromphenyl)-N-(thienyl-2-methyl)-amino]-2-imidazolin der Formel (I)

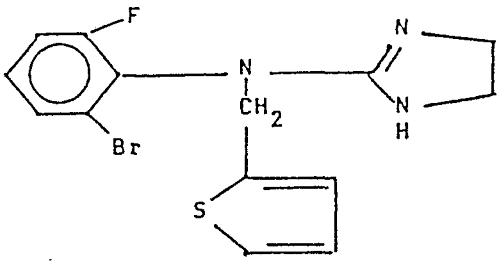

(I)

sowie dessen physiologisch verträgliche Säureadditionssalze mit wertvollen therapeutischen Eigenschaften.

Die Herstellung der Verbindung der Formel (I) erfolgt durch Umsetzung von 2-[(2-Fluor-6-bromphenyl)-imino]-imidazolidin der Formel (II)

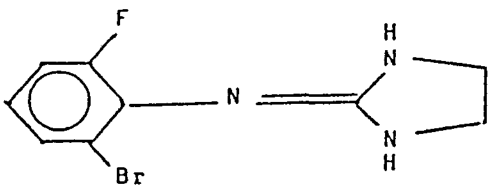

(II)

mit einem Halogenid der allgemeinen Formel (III)

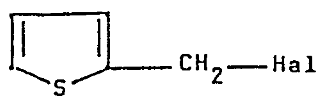

(III)

worin Hal ein Chlor-, Brom oder Jodatom bedeutet.

Die Umsetzung erfolgt durch Erhitzen der Reaktionspartner — vorzugsweise in Gegenwart eines polaren oder unpolaren Lösungsmittels — auf Temperaturen von 40 bis 150 °C.

Die speziellen Reaktionsbedingungen hängen in starkem Maße von der Reaktivität der Umsetzungsteilnehmer ab. Esempfiehlt sich, bei der Alkylierung das Halogenid der allgemeinen Formel (III) im Überschuß anzuwenden und die Umsetzung in Gegenwart eines säurebindenden Mittels durchzuführen.

Die Ausgangsverbindung der Formel (II) ist aus der BE-A-623 505 bekannt. Verbindungen der Formel (III) lassen sich durch Halogenierung der zugrundeliegenden Alkohole darstellen.

Das erfindungsgemäße 2-Phenylamino-imidazolin-(2) der Formel (I) kann auf übliche Weise in seine physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neue Verbindung der Formel (I) und deren Säureadditionssalze wirkt stark analgetisch und ist daher zur Behandlung von Schmerzzuständen geeignet. Die Verbindung der Formel (I) und deren Säureadditionssalze können enteral oder parenteral angewandt werden.

Die Dosierung liegt bei 0,1 bis 80 mg, vorzugsweise 1 bis 30 mg. Die Verbindung der Formel (I) bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung.

Beispiel 1

2-[N-(2-Fluor-6-brom-phenyl)-N-(thienyl-2-methyl)-amino]-2-imidazolin

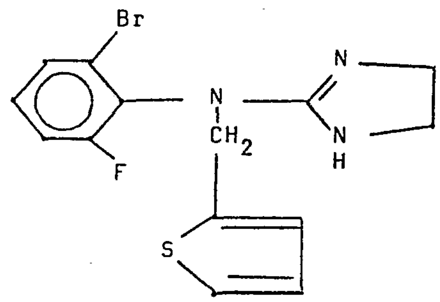

7,74 g (0,03 Mol) 2-[(2-Brom-6-fluor-phenyl)imino]imidazolidin werden zusammen mit 5 g (125 %) 2-Chlormethyl-thiophen und 4,5 ml Triäthylamin in 60 ml getrocknetem Toluol 6 Stunden lang unter Rühren am Rückfluß erhitzt. Der gebildete Niederschlag wird abgesaugt und in ca. 1 N HCL gelöst. Die salzsaure Lösung wird zweimal mit Äther extrahiert (Ätherextrakte werden verworfen) und anschließend mit 5 N NaOH alkalisiert. Das sich hierbei abscheidende Produkt wird abgesaugt, mit Wasser gewaschen und getrocknet.

Ausbeute : 6,5 g (61,2 % der Theorie)
Schmelzpunkt : 107-108 °C.
$C_{14}H_{13}BrFN_3S$ (354, 24)

| | | | | | | |
|---|---|---|---|---|---|---|
| Ber. : | C 47,47 | H 3,70 | N 11,86 | Br 22,56 | F 5,36 | S 9,04 |
| Gef. : | C 47,54 | H 3,91 | N 11,88 | Br 22,37 | F 5,24 | S 9,01 |

## Formulierungsbeispiele

Beispiel A : Dragées

| | |
|---|---|
| Wirkstoff gemäß Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

### Herstellung

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht gepreßt, die dann in üblicher Weise mit Hilfe von Zucker, Talkum und Gummi arabicum dragiert werden.

Beispiel B : Ampullen

| | |
|---|---|
| Wirkstoff gemäß Erfindung | 1,0 mg |
| Natriumchlorid | 18,0 mg |
| dest. Wasser | ad 2,0 ml |

### Herstellung

Wirkstoff und Natriumchlorid werden in Wasser gelöst und unter Stickstoff in Glasampullen abgefüllt.

Beispiel C : Tropfen

| | |
|---|---|
| Wirkstoff gemäß Erfindung | 0,02 g |
| p-Hydroxybenzoesäuremethylester | 0,07 g |
| p-Hydroxybenzoesäurepropylester | 0,03 g |
| entmineralisiertes Wasser | ad 100 ml |

**Ansprüche** (für die Vertragsstaaten : BE, CH, DE, FR, GB, IT, LI, LU, NL, SE)

1. 2-[N-(2-Fluor-6-bromphenyl)-N-(thienyl-2-methyl)-amino]-2-imidazolin der Formel (I)

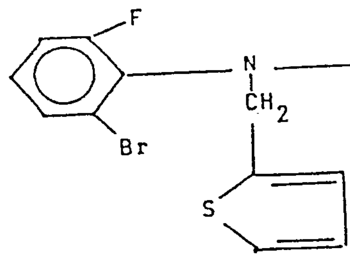

(I)

sowie dessen physiologisch verträgliche Säureadditionssalze.

2. Verfahren zur Herstellung der Verbindung en I nach Anspruch 1, dadurch gekennzeichnet, daß man 2-[(2-Fluor-6-bromphenyl)-imino]-imidazolidin der Formel (II)

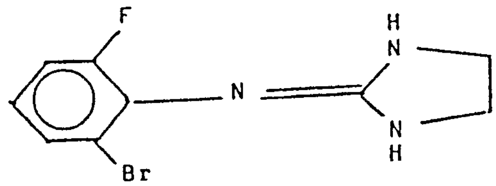

(II)

mit einem Halogenid der allgemeinen Formel (III)

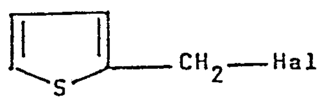

(III)

worin Hal ein Chlor-, Brom oder Jodatom bedeutet, in Gegenwart eines säurebindenden Mittels umsetzt, und daß man gegebenenfalls das nach dem Verfahren erhaltene End-produkt in ein Säureadditionssalz überführt.

3. Pharmazeutische Zubereitungen, dadurch gekennzeichnet, daß sie als Wirkstoff Verbindung en nach Anspruch 1 enthalten.

4. Verfahren zur Herstellung von Arzneimitteln nach Anspruch 3, dadurch gekennzeichnet, daß man die Wirkstoffe mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

5. Verbindungen nach Anspruch 1 zur Verwendung bei der Bekämpfung von Schmerzzuständen.

**Ansprüche** (für den Vertragsstaat AT)

1. Verfahren zur Herstellung von 2-[N-(2-Fluor-6-bromphenyl)-N-(thienyl-2-methyl)-amino]-2-imidazolin der Formel (I)

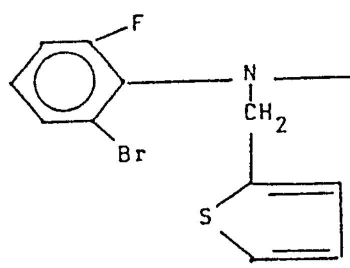

(I)

und von dessen physiologisch verträglichen Säureadditionssalzen, dadurch gekennzeichnet, daß man 2-[(2-Fluor-6-bromphenyl)-imino]-imidazolidin der Formel (II)

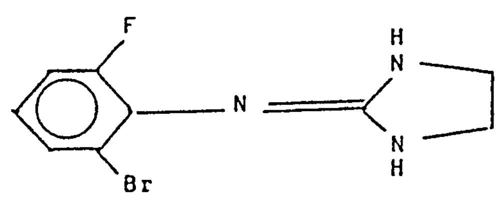

(II)

mit einem Halogenid der allgemeinen Formel (III)

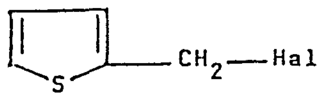

(III)

worin Hal ein Chlor-, Brom oder Jodatom bedeutet, in Gegenwart eines säurebindenden Mittels umsetzt, und daß man gegebenenfalls das nach einem Verfahren erhaltene Endprodukt in ein Säureadditionssalz überführt.

2. Verfahren zur Herstellung von Arzneimitteln, dadurch gekennzeichnet, daß man die Verbindung der Formel I oder deren Säureadditionssalze nach Anspruch 1 mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

**Claims** (for the Contracting States : BE, CH, DE, FR, GB, IT, LI, LU, NL, SE)

1. 2-[N-(2-fluoro-6-bromophenyl)-N-(thienyl-2-methyl)-amino]-2-imidazoline of formula (I)

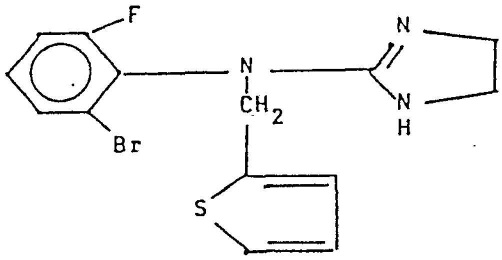

(I)

and its physiologically compatible acid addition salts.

2. Process for preparing the compounds according to claim 1, characterised in that 2-[(2-fluoro-6-bromophenyl)-amino]-imidazoline of formula (II)

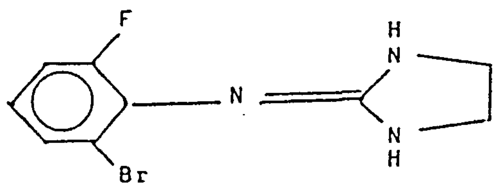

(II)

is reacted with a halide of general formula (III)

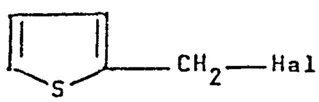

(III)

in which Hal represents a chlorine, bromine or iodine atom, in the presence of an acid-binding agent, and in that, if appropriate, the final product obtained according to the process is converted into an acid addition salt.

3. Pharmaceutical preparations, characterised in that they contain as active substance compounds according to claim 1.

4. Process for preparing pharmaceuticals according to claim 3, characterised in that the active substances are formulated with conventional galenic excipients, carriers, releasing agents or lubricants or substances for achieving a sustained release effect, into tablets, capsules, suppositories, solutions or powders.

5. Compounds according to claim 1 for use in controlling painful conditions.

**Claims** (for the Contracting State AT)

1. Process for preparing 2-[N-(2-fluoro-6-bromophenyl)-N-(thienyl-2-methyl)-amino]-2-imidazoline of formula (I)

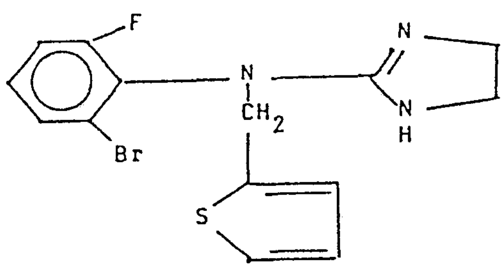

(I)

and its physiologically compatible acid addition salts, characterised in that 2-[(2-fluoro-6-bromophenyl)-amino]-imidazolidine of formula (II)

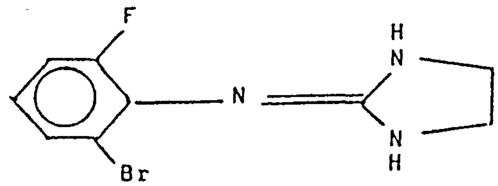

(II)

is reacted with a halide of general formula (III)

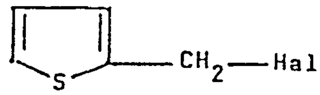

(III)

in which Hal represents a chlorine, bromine or iodine atom, in the presence of an acid-binding agent, and in that, if appropriate, the final product obtained according to a process is converted into an acid addition salt.

2. Process for preparing pharmaceuticals, characterised in that the compound of formula (I) or its acid addition salts according to claim 1 are formulated with conventional galenic excipients, carriers, releasing agents or lubricants or substances for achieving a sustained release effect, into tablets, capsules, suppositories, solutions or powders.

**Revendications** (pour les Etats contractants : BE, CH, DE, FR, GB, IT, LI, LU, NL, SE)

1. 2-[N-(2-fluoro-6-bromophényl)-N-(thiényl-2-méthyl)-amino]-2-imidazoline de formule (I)

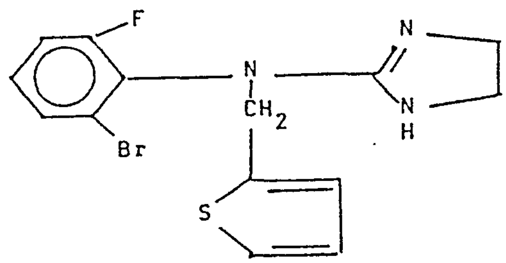

(I)

ainsi que ses sels d'addition d'acides physiologiquement supportables.

2. Procédé pour la préparation des composés selon la revendication 1, caractérisé en ce qu'on fait réagir la 2-[(2-fluoro-6-bromophényl)-imino]-imidazolidine de formule (II)

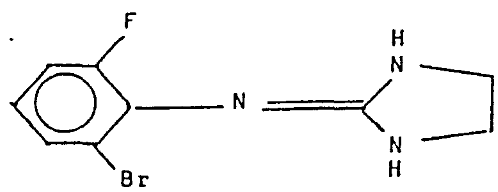

(II)

avec un halogénure de formule générale (III)

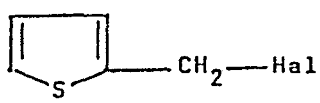

(III)

dans laquelle Hal représente un atome de chlore, de brome ou d'iode, en présence d'un agent fixant les acides, et en ce que éventuellement, on transforme le produit final obtenu selon le procédé en un sel d'addition d'acide.

3. Préparations pharmaceutiques, caractérisées en ce qu'elles contiennent en tant que substance active des composés selon la revendication 1.

4. Procédé pour la préparation de médicaments selon la revendication 3, caractérisé en ce qu'on formule les substances actives avec des adjuvants, excipients, désagrégeants ou lubrifiants ou respectivement substances pour obtenir un effet retard galéniques habituels en comprimés, capsules, suppositoires, solutions ou poudres.

5. Composés selon la revendication 1 pour l'utilisation dans la lutte contre les états douloureux.

**Revendications** (pour l'Etat contractant AT)

1. Procédé pour la préparation de la 2-[N-(2-fluoro-6-bromophényl)-N-(thiényl-2-méthyl)-amino]-2-imidazoline de formule (I)

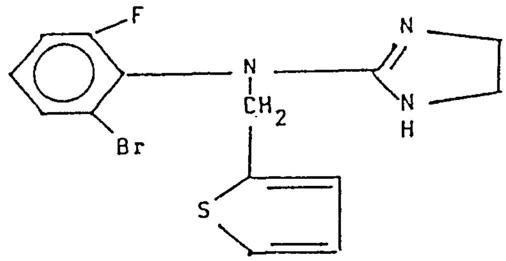

(I)

et de ses sels d'addition d'acides physiologiquement supportables, caractérisé en ce qu'on fait réagir la 2-[(2-fluoro-6-bromophényl)-imino]-imidazolidine de formule (II)

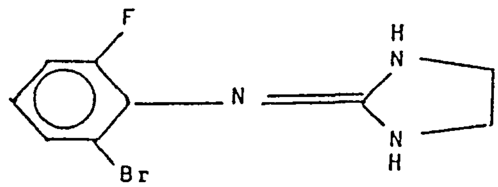

(II)

avec un halogénure de formule générale (III)

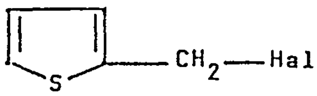

(III)

dans laquelle Hal représente un atome de chlore, de brome ou d'iode, en présence d'un agent fixant les acides, et en ce qu'éventuellement, on transforme le produit final obtenu selon le procédé en un sel d'addition d'acide.

2. Procédé pour la préparation de médicaments, caractérisé en ce qu'on formule le composé de formule (I) ou ses sels d'addition d'acides selon la revendication 1, avec des adjuvants, excipients, désagrégeants ou lubrifiants ou respectivement substances pour obtenir un effet retard galéniques habituels en comprimés, capsules, suppositoires, solutions ou poudres.